# EUROPEAN PATENT APPLICATION

(11) **EP 4 578 447 A1**
(43) Date of publication of application: **02.07.2025**
(21) Application number: 24223312.0
(22) Date of filing: 26.12.2024
(51) Int. Cl.: A61K 31/18, A61K 31/4196, A61K 31/4245, A61K 31/4439, A61K 31/495, A61K 31/513, A61K 31/5375, A61K 31/55, A61K 33/243, A61P 35/00

(54) **ERCC1-XPF COMPLEX INHIBITOR COMPOUNDS FOR USE IN THE TREATMENT OF CANCER**

(30) Priority: 27.12.2023 PT 2023119176
(71) Applicant: Fundação D. Anna de Sommer Champalimaud e Dr. Carlos Montez Champalimaud - Centro De Investigação Da Fundação Champalimaud, 1400-038 Lisboa (PT); FARM-ID - ASSOCIAÇÃO DA FACULDADE DE FARMÁCIA PARA A INVESTIGAÇÃO E DESENVOLVIMENTO, 1649-003 Lisboa (PT)
(72) Inventor: DE CASTILHO MONTEIRO GIL, NUNO JOSÉ, 1350-127 LISBOA (PT); DA ROCHA GUERREIRO DE OLIVEIRA, NUNO FILIPE, 2670-572 LOURES (PT); ÁLVARO MANGUINHAS, RITA CATARINA, 2910-433 SETÚBAL (PT); DO NASCIMENTO CARDOSO GUEDES, RITA ALEXANDRA, 1750-447 LISBOA (PT); ROSELL, RAFAEL, 08034 BARCELONA (ES); ALVES SERRA, PATRÍCIA FILIPA, 2695-622 SÃO JOÃO DA TALHA (PT)
(74) Representative: Monteiro Alves, Inês

(57) **Abstract**

The present invention discloses ERCC1-XPF complex inhibitor compounds and the use thereof in the treatment of cancer, in combination therapy or as standalone treatment. The invention further extends to pharmaceutical compositions that comprise an effective amount of these compounds, as well as kits comprising of the compounds and at least one anticancer agent, therefore providing a tailored approach for the treatment of specific cancers, such as non-small cell lung cancer.

## Description

[The present application claims the benefit of priority to the Portuguese provisional patent application no. 119176, filed on December 27, 2023, the contents of which is incorporated herein by reference.

### Technical Field

The present invention resides in the field of small-molecule organic compounds and encompasses a series of compounds which are identified for their therapeutic application as medicaments.

More specifically, the present invention discloses ERCC1-XPF complex inhibitors and the use thereof in the treatment of cancer, in combination therapy or as standalone treatment.

The scope of the invention also extends to pharmaceutical compositions that comprise an effective amount of these compounds, as well as kits consisting of the compounds and at least one anticancer agent, therefore providing a tailored approach for the treatment of specific cancers, such as non-small cell lung cancer.

### Background Art

Non-small cell lung cancer (NSCLC), the predominant subtype of lung cancer (LC), remains a significant global health challenge, contributing to a substantial proportion of cancer-related deaths. Despite significant advances in treatment strategies, many NSCLC patients continue to face reduced survival rates due to factors such as late diagnosis, metastatic progression, and both intrinsic and acquired resistance to therapies.

Cells undergo genomic instability at multiple stages of their life cycle. As a result of this instability, DNA lesions, either from endogenous or exogenous sources, are constantly formed. To preserve genomic integrity, cells have evolved a series of DNA repair pathways. While these pathways are essential for cellular health, cancer cells can exploit them for their benefit.

An increased expression of several genes involved in DNA repair mechanisms has been identified as biomarkers of cancer progression and has been associated with adverse clinical outcomes. Moreover, these repair pathways can also act as a double-edged sword for tumoral cells by providing a survival advantage when confronted with DNA-damaging agents, such as platinum-based drugs like cisplatin. This resistance to cisplatin has been associated with an increased DNA repair response in tumors, including NSCLC.

Particularly, the overexpression of the excision repair cross-complementation group 1 / xeroderma pigmentosum complementation group F (ERCC1-XPF) in the Nucleotide Excision Repair (NER) pathway has been linked to augmented repair of cisplatin-mediated DNA damage. Consequently, recent research has shifted towards targeting DNA repair pathways to enhance the efficacy of existing cancer treatments by sensitizing tumor cells to these therapeutic agents and potentially circumvent resistance.

This paradigm shift has given rise to an emerging cancer research field focusing on the synergistic use of DNA repair inhibitors with standard chemo/radiotherapy or as standalone treatments employing synthetic lethality. This approach, termed "DNA repair targeted therapy", has yielded promising drugs candidates, including poly(ADP-ribose) polymerase (PARP) inhibitors, some of which are in clinical trials or have received regulatory approval.

Modifications in DNA repair pathways are recognized as prognostic markers and potential therapeutic targets in various cancers. In the context of NSCLC, ERCC1 overexpression has been extensively investigated.

Elevated ERCC1 expression levels have pivotal implications for prognosis, therapeutic responses, and personalized treatment strategies. Given that ERCC1 overexpression is tied to suboptimal outcomes with cisplatin regimens, this protein emerges as a promising target for drug development in NSCLC.

The ERCC1 forms a heterodimer with XPF, forming the ERCC1-XPF complex, a critical component for DNA repair within the NER pathway. While ERCC1 modulates various DNA and protein interactions, the endonuclease activity is attributed to XPF, which also harbors a non-functional helicase-like motif implicated in protein-protein interactions and DNA binding.

Prior computational studies aimed to identify potential inhibitors of this complex, shedding light on important structural features vital for its function. Initial attempts at inhibiting this complex focused on disrupting the interaction between Xeroderma Pigmentosum group A (XPA) and ERCC1, with XPA playing a role in recruiting ERCC 1-XPF to DNA damaged sites within the NER pathway.

While certain inhibitors showed promising results, they lacked specificity for other processes in which ERCC1-XPF, but not XPA, is involved, such as interstrand cross-link repair (ICL repair) and DNA double-strand break (DSB) repair. Other authors have targeted the interactions between XPF and DNA and between XPF and ERCC1, but many of these molecules have issues related to off-target cytotoxicity, poor pharmacokinetics, and reduced potency.

Therefore, a need exists in the art for finding novel compounds useful as inhibitors for the ERCC1-XPF complex, especially those which are able to enhance cisplatin activity. Such compounds shall be simple small molecules with good spatial fitting in the ERCC1-XPF active binding site and establish important interactions with residues essential for complex formation. The present invention satisfies this need.

### Prior art documents

It is already disclosed in the prior art that the ERCC1-XPF complex is a novel and potential therapeutic target to overcome chemoresistance in cancer therapy and the prior art documents collectively highlight the ongoing efforts to develop effective ERCC1-XPF inhibitors that can enhance the efficacy of cancer therapies.

Early studies have identified compounds that inhibit ERCC1-XPF endonuclease activity *in vitro*: 2,7-naphthalenedisulfonic acid compounds lacked specificity and had limited cellular activity and monoclonal antibodies targeting ERCC1 like emeleximab are limited to experimental settings due to delivery challenges *in vivo.*

Prior art document CN111298122B discloses a pharmaceutical composition for the treatment of small cell lung cancer (SCLC) comprising lurbinectedin and a selective norepinephrine reuptake inhibitor, such as reboxetine. Significant synergistic effects were found for the combination of lurbinectedin (a DNA-alkylating chemotherapy agent) and the antidepressant maprotiline and no combinations of the drugs with cisplatin were considered.

In article *"Computational characterization of small molecules binding to the human XPF active site and virtual screening to identify potential new DNA repair inhibitors targeting the ERCCI-XPF endonuclease",* the authors adopted a homology modeling strategy to build a structural model of the human XPF nuclease domain that contained the active site and to also extract dominant conformations using molecular dynamics simulations followed by trajectory clustering.

In this context, small inhibitor molecules were investigated, with the aim of building the pharmacophore model after molecular docking simulations targeting the XPF domain. Despite the chemical similarities (such as the inclusion of at least one metal binding motif), the compounds disclosed are distinct from the compounds useful as inhibitors for the ERCC 1-XPF complex of the present invention.

As shown, current ERCC1-XPF inhibitors face several limitations, such as lack of specificity for ERCC 1-XPF and non-specific DNA repair inhibition, which leads to undesirable side effects by affecting normal cells.

Thus, it is an object of the present invention to find compounds which are useful as inhibitors for the ERCC 1-XPF complex in a specific way, therefore reducing off-target effects and minimizing toxicity to normal cells.

### Summary of Invention

In a first aspect of the present invention, it is disclosed a series of ERCC1-XPF complex inhibitors compounds and / or a pharmaceutically acceptable salt, solvate or tautomer thereof for use as a medicament in the treatment of cancer, tumors and / or metastases.

In a second aspect of the present invention, it is disclosed a pharmaceutical composition comprising an effective amount of at least one ERCC1-XPF complex inhibitor compound and / or a pharmaceutically acceptable salt, solvate or tautomer thereof and excipients.

In a third aspect of the present invention, it is disclosed a kit consisting of separate packs of (a) an effective amount of a ERCC1-XPF complex inhibitor compound and / or a pharmaceutically acceptable salt, solvate or tautomer thereof, and (b) an effective amount of at least one anticancer agent.

### Technical Problem

The ERCC1-XPF is a protein complex that plays a critical role in repairing damaged DNA and maintaining genomic stability. Both proteins ERCC1 and XPF are necessary for the complex to function, wherein XPF provides the ability to cut DNA, while ERCC1 stabilizes the complex and helps it recognize and bind to specific DNA structures.

Since ERCC1-XPF recognizes and acts on specific DNA structures (e.g., single-strand / double-strand junctions, bubble-like distortions, or crosslinks) and not on specific sequences or the cell type, the complex is not specific to normal cells or cancer cells - it is a general DNA repair complex that functions wherever it is expressed, repairing DNA damage in all cells that produce it, whether they are healthy, cancerous, or otherwise abnormal.

Cancer cells often experience high levels of DNA damage due to their rapid proliferation, genomic instability, and exposure to both endogenous and exogenous stressors, such as chemotherapeutic agents and radiation therapies that induce DNA damage to trigger apoptosis in cancer cells.

Cancer cells with upregulated ERCC1-XPF activity can efficiently repair this therapy-induced damage, allowing cancer cells to continue proliferating despite the therapeutic assault. Thus, inhibiting ERCC1-XPF activity could sensitize cancer cells to DNA-damaging agents, increasing the efficacy of chemotherapy and radiation therapy.

### Solution to Problem

The present invention discloses novel and more efficient ERCC1-XPF inhibitors for use thereof in the treatment of cancer, in combination therapy or as standalone treatment.

To this end, the inventors implemented a structure-based virtual screening (SBVS) strategy, emphasizing the disruption of ERCC1 and XPF interactions, and then characterized the interface domain using crystallographic data. Molecular docking and virtual screening calculations were performed and subsequently used to screen millions of compounds, which were then selected based on their score, molecular weight, lipophilicity, ligand efficiency, appropriate pocket fit, and interactions with nearby residues. At last, the selected compounds underwent validation in cell-based *in vitro* assays with a NSCLC cell line with higher ERCC1 expression, and in combination with cisplatin.

The scope of the invention also extends to pharmaceutical compositions that comprise an effective amount of these compounds, as well as kits consisting of the compounds and at least one anticancer agent, providing a tailored approach for the treatment of specific cancers.

### Advantageous Effects of Invention

By inhibiting ERCC1-XPF-mediated DNA repair, the compounds of the present invention increase the sensitivity of cancer cells to DNA-damaging agents, making them more susceptible to DNA damage-induced apoptosis.

Since the compounds can be used in combination with various anticancer agents, their applicability across different chemotherapy regimens is broadened, leading to improved treatment outcomes.

Furthermore, the compounds are designed to specifically target the ERCC1-XPF complex, potentially reducing off-target effects and minimizing toxicity to normal cells.

### Brief Description of Drawings

In order to facilitate an understanding of the principles according to the embodiments of this invention, reference will be made to the illustrated embodiments in the Figures and the language used to describe them.

It should also be understood that there is no intention to limit the scope of the invention to the content of the Figures and that modifications to the inventive features illustrated herein, as well as additional applications of the illustrated principles and embodiments, which would normally occur to a person skilled in the art having possession of this description, are considered within the scope of the claimed invention.
**Fig.1**

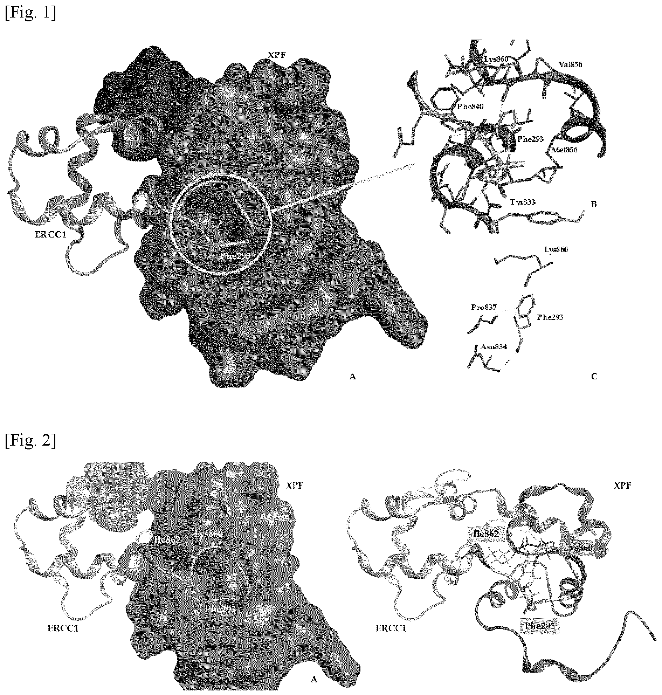

   [Fig.1] depicts the ERCC1-XPF complex structure with emphasis on the binding site and its interactions wherein (A) is the ERCC1-XPF complex with emphasis on the XPF pocket enveloping Phe293 from ERCC1, based on the 1Z00 pdb structure, (B) is a close-up of the pocket region revealing Phe293 (highlighted) forming a hydrogen bond with Lys860 of XPF and participating in aromatic interactions with both Pro837 and Asn834; and (C) is an in-depth perspective of the XPF residues interacting with Phe293.
**Fig.2**
   [Fig.2] depicts (A) the ERCC1-XPF complex highlighting the XPF pocket formed around Phe293 from ERCC1, (B) a close-up view of the pocket region showcases Phe293 from ERCC1 (highlighted) and the two residues Leu860 and Ile862 from XPF.
**Fig.3**

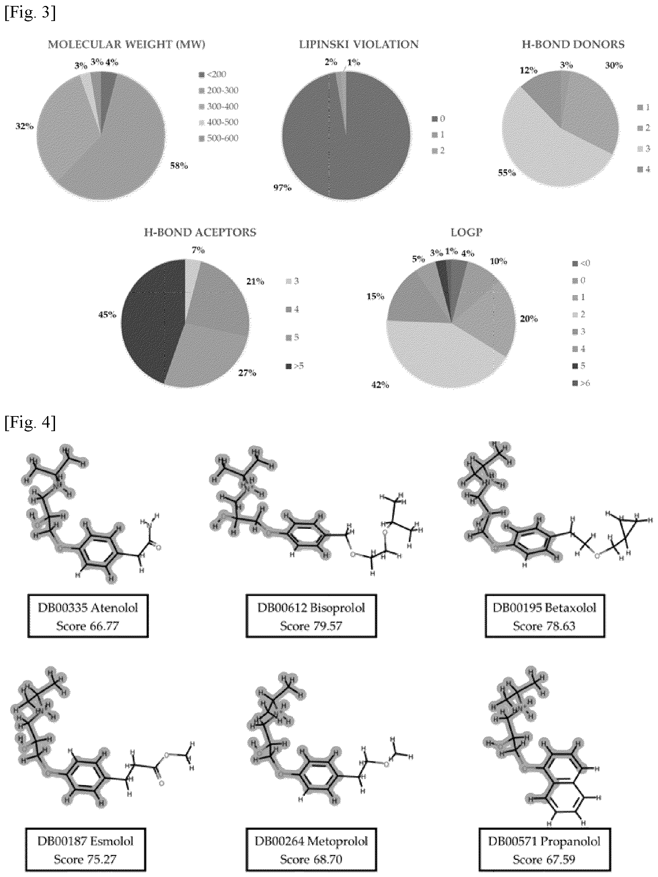

   [Fig.3] depicts a graphical representation of the distribution of molecular descriptors of the molecules retrieved from ChEMBL with activities up to 30 µM, the descriptors including molecular weight (MW), Lipinski violations, H-bond donors, H-bond acceptors, and LogP.
**Fig.4**
   [Fig.4] is a representation of Cluster 1 derived from the DrugBank results dataset proposed from the visual inspection from a total of 48 molecules.
**Fig.5**

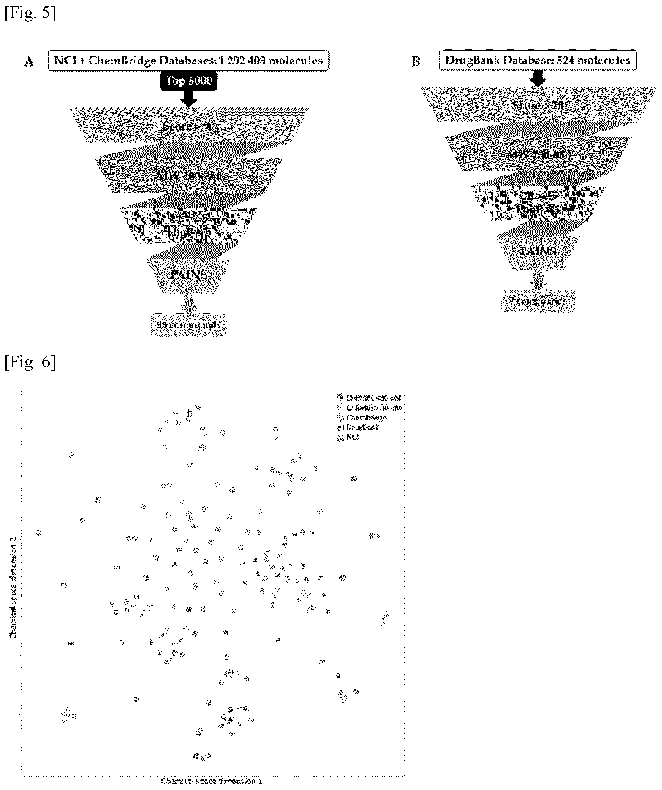

   [Fig.5] is a schematic representation of the filtering criteria applied for compound selection from the selected databases, wherein (A) includes compounds from both the NCI and ChemBridge databases, and (B) includes compounds from the DrugBank database.
**Fig.6**
   [Fig.6] depicts the t-SNE distribution representing the chemical diversity of active and inactive compounds from ChEMBL.
**Fig.7**

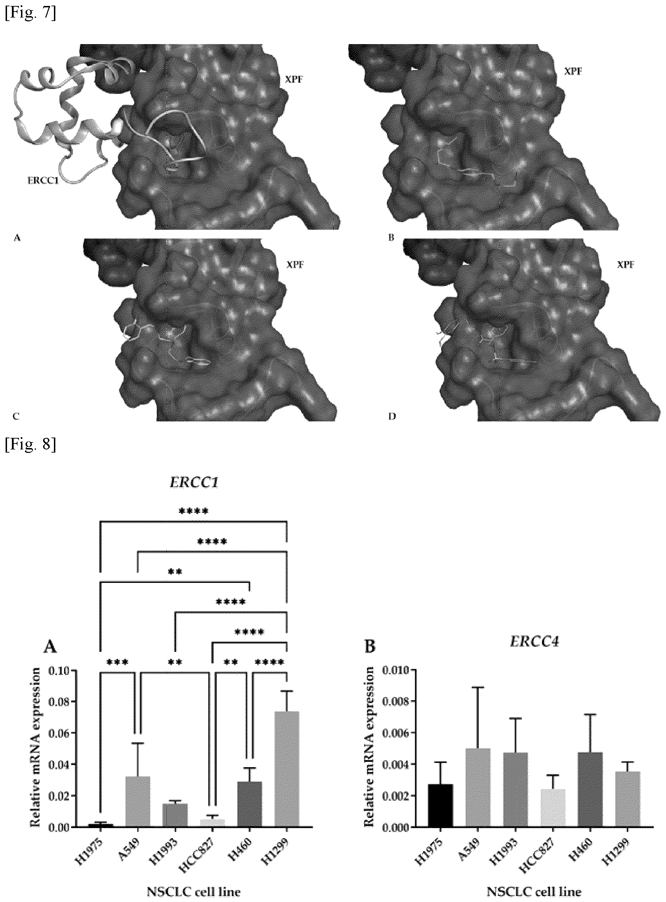

   [Fig.7] is a representation of compounds docked into the XPF protein binding pocket, wherein (A) is the ERCC1-XPF complex, highlighting the Phe293 residue from ERCC1 located inside the binding pocket. Docked complexes of XPF with potential inhibitors selected from (B) DrugBank, (C) NCI database, and (D) ChemBridge database. All these molecules fit snugly within the pocket, effectively obstructing its access.
**Fig.8**
   [Fig.8] shows relative mRNA expression levels (β-actin) of genes ERCC1 and ERCC4 for all the NSCLC studied cell lines, wherein total RNA was isolated from six NSCLC cell lines and gene expression was analyzed by qRT-qPCR for (A) ERCC1 and (B) ERCC4 genes. Results are expressed as mean ± SD (n=3). **p < 0.01, ***p < 0.001, and ****p < 0.0001 (one-way ANOVA, Tukey's multiple comparisons test).
**Fig.9**

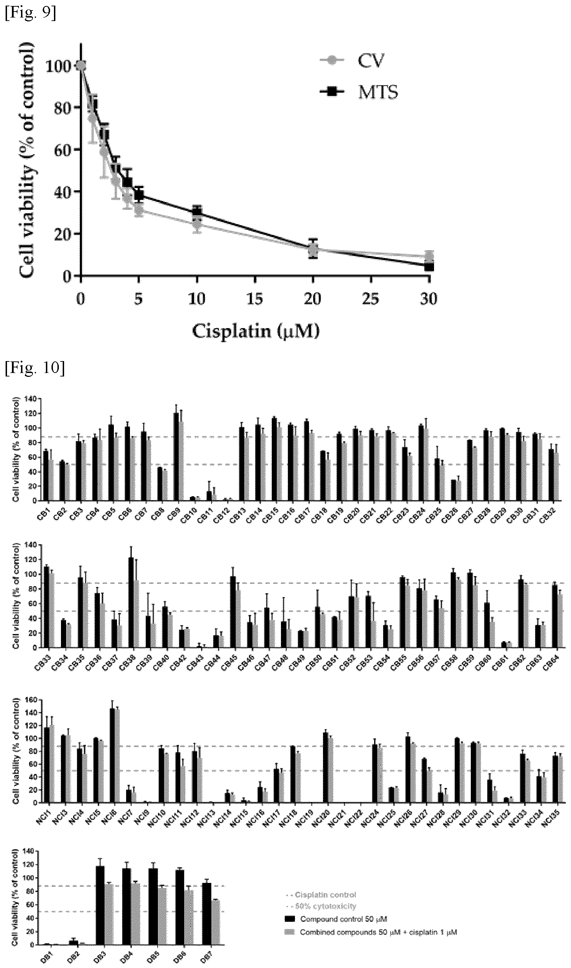

   [Fig.9] shows cytotoxic effects of cisplatin in H1299 cells treated for 72 h. Values represent mean ± SD and are expressed as percentages of the vehicle-treated control cells.
**Fig.10**
   [Fig.10] shows the assessment of the sensitizing effect of the putative ERCC1-XPF inhibitors in terms of cisplatin-induced cytotoxicity. An MTS assay was performed, and all compounds (50 µM) selected from (A,B) ChemBridge, (C) NCI, and (D) DrugBank databases were incubated with cisplatin (1 µM) for 72 h. Values represent mean ± SD and are expressed as percentages of the vehicle-treated control cells.
**Fig.11**

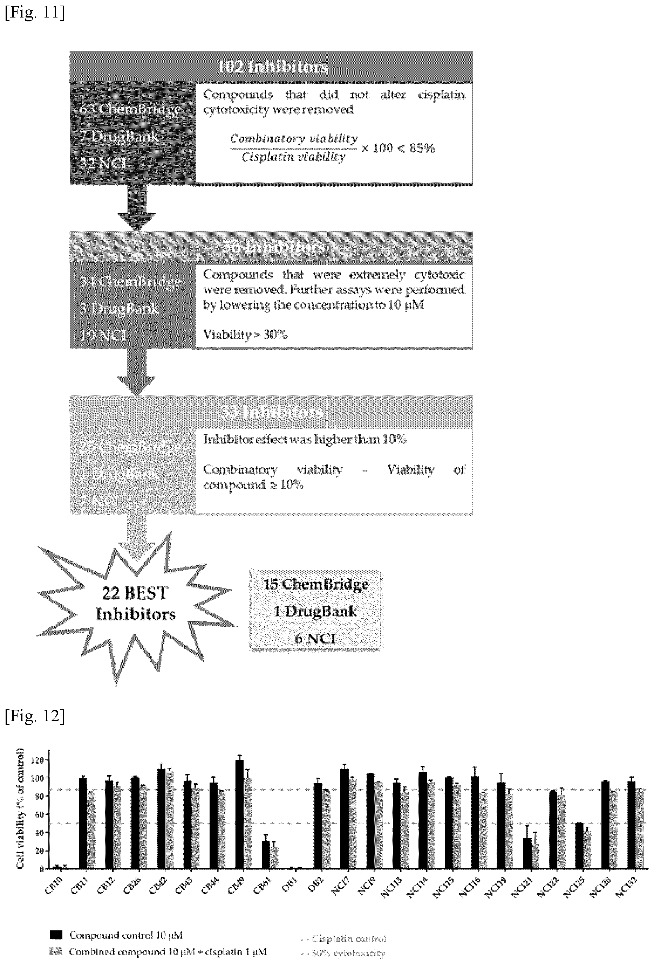

   [Fig.11] depicts the strategy employed to achieve the 22 best inhibitors.
**Fig.12**
   [Fig.12] shows the evaluation of the sensitizing effect of ERCC1-XPF inhibitors, which were considered extremely cytotoxic at 50 µM, in cisplatin-induced cytotoxicity. An MTS assay was performed in H1299 cells, and all compounds (10 µM) were incubated with cisplatin (1 µM) for 72 h. Values represent mean ± SD and are expressed as percentages of the vehicle-treated control cells.
**Fig.13**

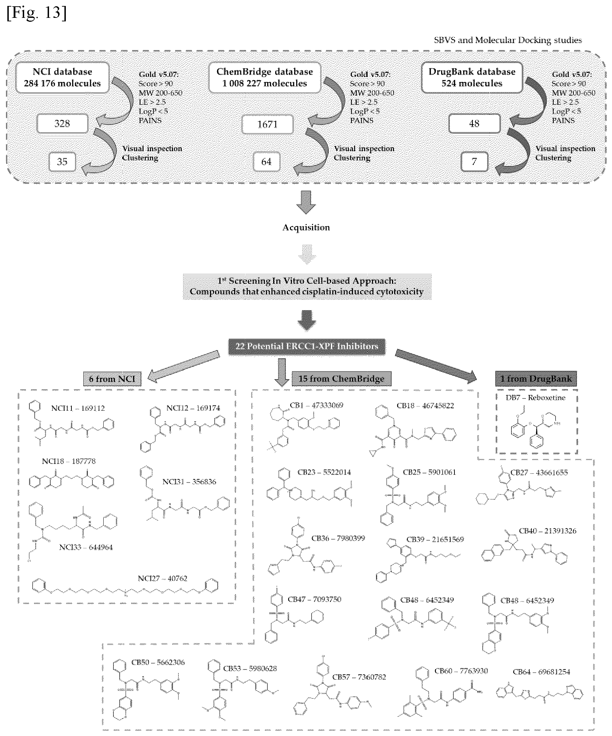

   [Fig.13] depicts a schematic decision-making process for the selection of the 22 best inhibitors.
**Fig.14**

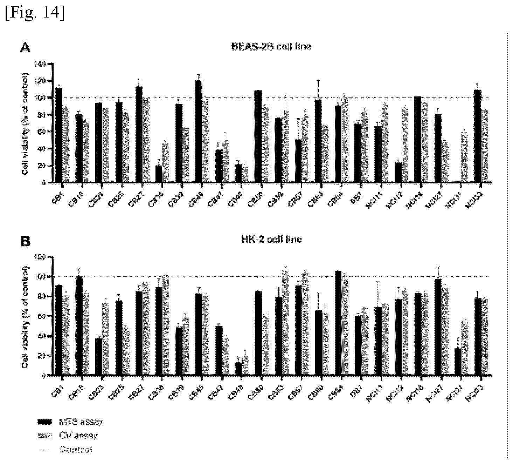

   [Fig.14] shows the assessment of the cytotoxic effects of putative ERCC1-XPF inhibitors in human non-tumoral cell lines of lung (BEAS-2B) and kidney (HK-2). MTS and CV assays were performed for all the best 22 compounds (50 µM, 72 h incubation) on the (A) BEAS-2B and (B) HK-2 cell lines. Values represent mean ± SD and are expressed as percentages of the vehicle-treated control cells.

### Description of Embodiments

The present invention discloses, in a first aspect, ERCC1-XPF complex inhibitors compounds that work by destabilizing the ERCC1-XPF complex.

Components of the NER pathway, namely the ERCC1-XPF complex, have emerged as druggable targets with important implications in cancer therapy. Several studies demonstrated that interfering with the complex function could improve standard platinum-based chemotherapy.

Analysis of crystal structures and a library of small molecules known to act against the complex highlighted the pivotal role of Phe293 (ERCC1) in maintaining complex stability. Thus, this residue was chosen as the primary binding site for virtual screening.

In the present invention, novel molecules for ERCC1-XPF inhibition were identified. The selection of a given compound to proceed in further biological tests was based on a combination of general performance considering the score, the molecular weight, lipophilicity, ligand efficiency, and the appropriate pocket fitting and interactions with the nearby residues. Thus, the ERCC1-XPF complex inhibitors compounds of the present invention comprise:
at least one optionally substituted cyclyl, heterocyclyl, aryl or heteroaryl group;
from 2 to 3 hydrogen bond donors selected from at least one of a hydroxyl group, a thiol group, a fluoride group and a nitrogen-containing group;
at least 4 hydrogen bond acceptors selected from at least one of oxygen, nitrogen, sulfur and halogens;
wherein the molecular weight ranges between 200 and 400 g/mol;
and / or a pharmaceutically acceptable salt, solvate or tautomer thereof for use as a medicament in the treatment of cancer, tumors and / or metastases, wherein the compound and the Phe293 residue from ERCC1 participate in a molecular interaction to destabilize the ERCC1-XPF complex.

In a more preferred embodiment of the present invention, the ERCC1-XPF complex inhibitors compounds comprise:
at least one optionally substituted phenyl, furanyl, pyrrolyl, thienyl, oxazolyl, indazolyl, isoxazolyl, thiazolyl, isothiazolyl, imidazolyl, pyrazolyl, triazolyl, pyridyl, pyridinyl, dihydrothiadiazolyl, dioxazolyl, oxadiazolyl, pyrazinyl, pyrimidinyl, pyridazinyl, piperazinyl, triazinyl, indolyl, quinolinyl, isoquinolinyl, purpyrrolyl, benzimidazolyl, benzoxazolyl, benzothiazolyl, pteridinyl, azepanyl, diazinanyl, pyrrolidinyl, imidazolidinyl, morpholinyl or other mono- or poly aromatic or non-aromatic homocyclic or heterocyclic compound;
from 2 to 3 hydrogen bond donors selected from at least one of a hydroxyl group, a thiol group, a fluoride group and a nitrogen-containing group selected from substituted or unsubstituted amino group selected from primary or secondary amine or amide;
at least 4 hydrogen bond acceptors selected from at least one of oxygen, nitrogen, sulfur and halogens, the oxygen being found in carbonyl groups, hydroxyl groups, ether groups or esters groups, the nitrogen being found in amines, amides and heteroaryl groups, and the sulfur being found in sulfonyl and thiocarbonyl groups;
wherein the molecular weight ranges between 200 and 400 g/mol;
and / or a pharmaceutically acceptable salt, solvate or tautomer thereof for use as a medicament in the treatment of cancer, tumors and / or metastases.

An initial screening approach was developed *in vitro* and comprised the identification of compounds that could in fact enhance cisplatin-induced cytotoxicity in NSCLC cell lines with high ERCC1 expression.

Of all the tested compounds, 22 of them demonstrated positive results in enhancing cisplatin-induced cytotoxicity. All these compounds are simple small molecules with good spatial fitting in the ERCC1-XPF active binding site and establish important interactions with residues essential for complex formation.

In a preferred embodiment of the present invention, the ERCC 1-XPF complex inhibitor compounds are selected from:

### N-{3-methoxy-4-[2-(2-pyridinyl)ethoxy]benzyl}-N-[(3 S)-2-oxo-3-azepanyl]-2-[3-(trifluoromethyl)phenyl]acetamide

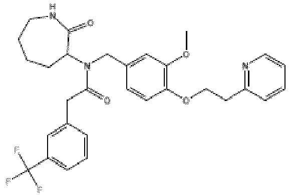

### 1-benzyl-N'-cyclopropyl-N-methyl-4-oxo-N-[(4-phenyl-1,3-thiazol-2-yl)methyl]-1,4-dihydro-3,5-pyridinedicarboxamide

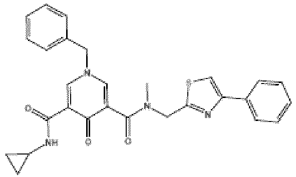

### 1-[(3,4-dimethoxybenzyl)oxy]-3-[4-(diphenylmethyl)-1-piperazinyl]-2-propanol dihydrochloride

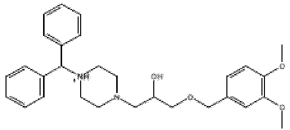

### N~2~-benzyl-N~1~-[2-(3,4-dimethoxyphenyl)ethyl]-N~2~-[(4-methoxyphenyl)sulfonyl]glycinamide

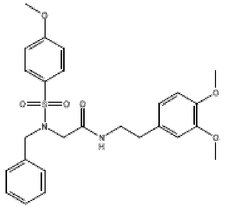

### N-{[5-[(cyclohexylmethyl)thio]-4-(4-fluorophenyl)-4H-1,2,4-triazol-3-yl]methyl}-3-(3-methyl-1H-pyrazol-1-yl)propenamide

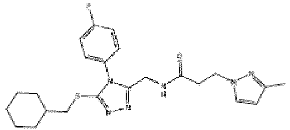

### 2-{1-(4-chlorophenyl)-5-oxo-3-[2-(2-thienyl)ethyl]-2-thioxo-4-imidazolidinyl}-N-(4-fluorophenyl)acetamide

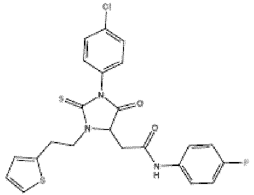

### N-(3-ethoxypropyl)-2-[2-[(4-phenyl-1-piperazinyl)methyl]-4-(3-thienyl)phenoxy]acetamide

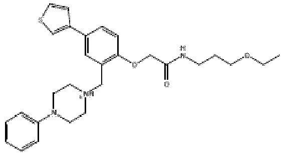

### 3-[2-(2-naphthylmethyl)-5-oxo-2-pyrrolidinyl]-N-[1-(1-phenyl-1H-pyrazol-4-yl)ethyl]propenamide

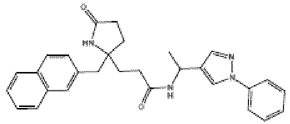

### N~2~-benzyl-N~1~-[2-(1-cyclohexen-1-yl)ethyl]-N~2~-[(4-fluorophenyl)sulfonyl]glycinamide

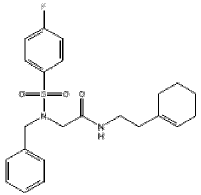

### N~2~-benzyl-N~2~-[(4-fluorophenyl)sulfonyl]-N~1~-[3-(trifluoromethyl)phenyl]glycinamide

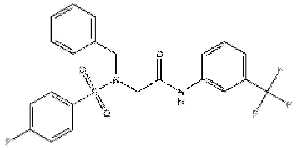

### N-2--benzyl-N-2--(2,3-dihydro-1,4-benzodioxin-6-ylsulfonyl)-N-1--[2-(3,4-dimethoxyphenyl)ethyl]glycinamide

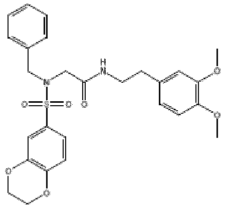

### N~2~-benzyl-N~2~-[(3,4-dimethoxyphenyl)sulfonyl]-N~1~-[2-(4-methoxyphenyl)ethyl]glycinamide

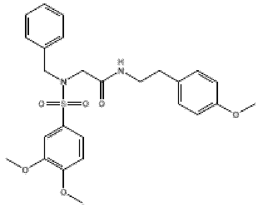

### 2-{1-(4-chlorophenyl)-5-oxo-3-[2-(4-pyridinyl)ethyl]-2-thioxo-4-imidazolidinyl}-N-(4-methoxyphenyl)acetamide

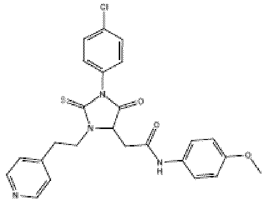

### 4-{[N-(mesityl sulfonyl)-N-(2-phenylethyl)glycyl]amino}benzamide

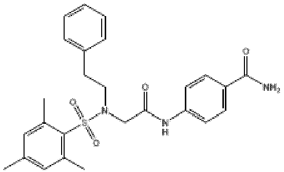

### N-[3-(1H-indazol-1-yl)propyl]-3-[5-(1H-indol-3-ylmethyl)-1,3,4-oxadiazol-2-yl]propenamide

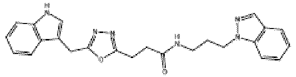

### (2R)-2-[(R)-(2-ethoxyphenoxy)-phenylmethyl]morpholine

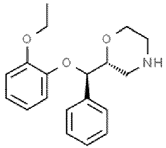

### Benzyl 4-methyl-2-[[2-[[2(phenylmethoxycarbonylamino)acetyl]amino]acetyl]amino]pentanoate

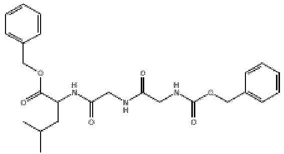

### Benzyl 3-phenyl-2-[[2-[[2-(phenylmethoxycarbonylamino)acetyl]amino]acetyl]amino]propanoate

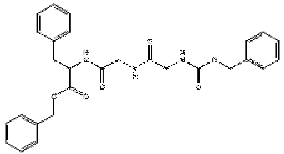

### 3-Benzyl-1 -[3-(3-benzyl-4-oxo-2-sulfanylidene-1,3-diazinan-1 -yl)propyl]-2-sulfanylidene-1,3-diazinan-4-one

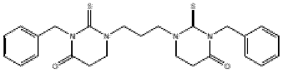

### Carbobenzyloxyvalylglycylglycine benzyl ester

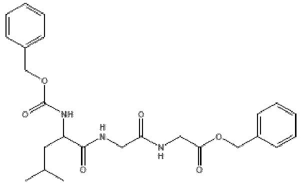

### 2-acetamido-N-benzyl-6-[benzyl(2-chloroethylcarbamoyl)amino]hexanamide

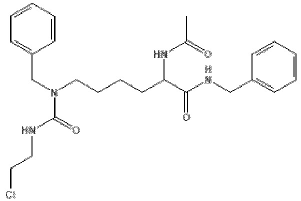

### 2-[2-[2-(2-phenoxyethoxy)ethoxy]ethoxy]-N-[2-[2-[2-(2-phenoxyethoxy)ethoxy] ethoxy] ethyl] ethanamine

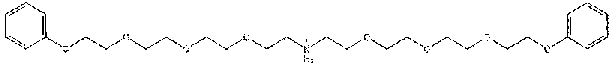

and / or a pharmaceutically acceptable salt, solvate or tautomer thereof for use as a medicament in the treatment of cancer, tumors and / or metastases, wherein the compound and the Phe293 residue from ERCC1 participate in a molecular interaction to destabilize the ERCC1-XPF complex.

In preferred embodiments of the present invention, the ERCC1-XPF complex inhibitors compound and / or pharmaceutically acceptable salt, solvate or tautomer thereof is used in combination with at least one anticancer agent. In more preferred embodiments of the present invention, the anticancer agent is a platinum-based anticancer agent.

In more preferred embodiments of the present invention, the anticancer agent is a platinum-based anticancer agent selected from the group consisting of cisplatin, carboplatin, oxaliplatin, nedaplatin, lobaplatin, satraplatin, and heptaplatin.

In preferred embodiments of the present invention, the ERCC 1-XPF complex inhibitors compound and / or pharmaceutically acceptable salt, solvate or tautomer thereof is used in the treatment of non-small cell lung cancer.

In a second aspect of the present invention, it is disclosed a pharmaceutical composition comprising an effective amount of at least one ERCC1-XPF complex inhibitors compound or a pharmaceutically acceptable salt, solvate or tautomer thereof and excipients. In preferred embodiments of the present invention, the pharmaceutical composition further comprises at least one anticancer agent, wherein the anticancer agent is a platinum-based anticancer agent selected from the group consisting of cisplatin, carboplatin, oxaliplatin, nedaplatin, lobaplatin, satraplatin, and heptaplatin.

In a third aspect of the present invention, it is disclosed a kit comprising of separate packs of
(a) an effective amount of a ERCC1-XPF complex inhibitor compound and / or a pharmaceutically acceptable salt, solvate or tautomer thereof, and
(b) an effective amount of at least one anticancer agent, wherein the anticancer agent is a platinum-based anticancer agent selected from the group consisting of cisplatin, carboplatin, oxaliplatin, nedaplatin, lobaplatin, satraplatin, and heptaplatin.

Overall, the present invention illustrates the importance of addressing small-molecule key features to guide the discovery of modulators of ERCC1-XPF interaction, providing important insights leading to the identification of potential inhibitors with novel chemotypes to be used in combination with platinum-based therapy in cancer, more specifically, cisplatin therapy in non-small cell lung cancer.

### Examples

In the present invention, a structure-based virtual screening strategy targeting the inhibition of ERCC1 and XPF interaction was developed. Using the optimized docking protocol described below, the compounds were screened and their capability to amplify cisplatin-induced cytotoxicity was assessed in NSCLC H1299 cells, which exhibited higher ERCC1 expression.

### Computational studies on the ERCC1-XPF Protein Complex

The ERCC1-XPF heterodimer serves as a structure-specific 5'-3' endonuclease, essential for DNA damage repair via the NER pathway. While ERCC1 is catalytically inactive, it plays a vital role in modulating DNA-protein and protein-protein interactions. The endonuclease activity, on the other hand, is provided by XPF. Additionally, XPF features an inactive helicase-like motif, which is believed to participate in protein-protein interactions and DNA binding.

A structure-based virtual screening (SBVS) strategy was adopted, which encompassed a comprehensive structural and physicochemical analysis and characterization of the ERCC1-XPF complex. Subsequently, a library of small molecules known to exhibit activity against the complex was refined.

This approach is critical for gathering crucial information on structural properties, binding regions, flexibility, key binding pocket interactions, and the dynamics of ligand-receptor interactions.

### Structural Analysis of the Protein Complex

Understanding structural properties, binding regions, flexibility, key interactions within binding pockets, and dynamics of ligand-receptor interactions are essential for drug development.

To achieve a comprehensive structural analysis and characterization of the ERCC1-XPF complex, available three-dimensional (3D) structures were explored. The 3D structures of the ERCC1-XPF complex which had been published and made available in the Protein Data Bank (PDB) are disclosed in Table 1. It is worth noting that these structures pertain to the catalytic domain and none of them had bound a small-molecule inhibitor.

**Table 1. Compilation of experimental 3D structures available for the ERCC1-XPF complex in the Protein Data Bank (PDB). Structures shaded in gray were used for subsequent analyses.**

| **PDB id** | **Res. (Å)** | **Year** | **Complex** | **Ref.** |
|---|---|---|---|---|
| 6SXA | 3.60 | 2020 | ERCC1-XPF Cryo-EM structure, Apoform | Jones et al. 2020 |
| 6SXB | 7.90 | 2020 | ERCC1-XPF Cryo-EM structure, DNA-bound form | Jones et al. 2020 |
| 2MUT | Solution | 2015 | Solution structure of the F231L mutant ERCC1-XPF dimerization region | Faridounnia et al. 2015 |
| 2KN7 | Solution | 2010 | Structure of the XPF-single strand DNA complex | Das et al. 2012 |
| 2AQ0 | Solution | 2006 | Solution structure of the human homodimeric DNA repair protein XPF | Das et al. 2007 |
| 2A1J | 2.70 | 2005 | Crystal Structure of the Complex between the C-Terminal Domains of Human XPF and ERCC1 | Tsodikov et al. 2005 |
| 2A1I | 1.90 | 2005 | Crystal Structure of the Central Domain of Human ERCC 1 | Tsodikov et al. 2005 |
| 1Z00 | Solution | 2005 | Solution structure of the C-terminal domain of ERCC1 complexed with the C-terminal domain of XPF | Tripsianes et al. 2005 |

In a first analysis, the retrieved 3D structures of ERCC1 and XPF proteins with PDBIDs 1Z00, 2A1J, 6SXA and 6SXB were aligned and superimposed. These proteins contain double helix-hairpin-helix (HhH2) motifs, crucial for dimerization. The superimposition unveiled significant similarity, particularly in the vicinity of the putative binding pocket region.

This region is defined around Phe293 of ERCC1, as identified using Molecular Operation Environment (MOE) software version v.2020.0901. The root means square deviations (RMSD) ranged from 1.30 Å for ERCC1 to 1.60 Å for XPF. A careful analysis of the complex highlighted that the pocket enveloping Phe293 of ERCC1 is highly conserved, suggesting its potential as a key site for inhibiting the complex's activity. The aromatic ring of Phe293 fits within the hydrophobic cavity of the XPF protein, defining a pocket with a large contact surface area of 280 Å 2, as calculated using MOE (Figure 1).

This structural analysis unveils clear interactions between the two proteins within the complex. The ERCC1 backbone forms crucial interactions with XPF, establishing three hydrogen bonds that anchor the side chain of Phe293 within the pocket. As depicted in Figure 1, the interactions involving Phe293 of ERCC1, and residues Asn834, Pro837, and Lys860 of XPF play a significant influence in stabilizing the complex.

Figure 1 further illustrates the interaction between the C-terminal regions of ERCC1 and XPF, leading to heterodimer formation, facilitated by the HhH2 motifs present in both proteins. The activity domain of ERCC1 adopts its functional conformation only alongside XPF, serving as a foundation for the proper folding of ERCC1. When isolated, both structures are prone to degradation, lacking structural stabilization. Notably, the deletion of the single residue Phe293 from ERCC1 is sufficient to stop the dimerization process between ERCC1 and XPF. This is anticipated given its strategic position within the binding pocket region. The residue Phe840 in XPF is also pivotal for nonbonded interactions within the complex, as its aromatic ring interacts with several ERCC1 residues (Figure 1B). Additional important features are evident in the interface region, including the pocket's encapsulation by the side chains of Tyr833 from XPF and Leu294 from ERCC1, both interacting with Phe293 located within the pocket.

Among the four structures analyzed, the 1Z00 pdb structure contains the highest number of residues in the pocket's critical region.

The pocket analysis indicates that the residues Ile862 and Lys860 from XPF face the pocket, suggesting potential guiding capabilities for small molecules. Specifically, Ile862 was selected due to its location on the inner side of the pocket, implying its potential to guide molecules into the pocket.

Consequently, these residues were selected and evaluated as potential central residues for defining the center of the docking box (binding site) in subsequent calculations (Figure 2 A-B). The generated binding pocket predominantly possesses hydrophobic properties, characterized by its significant depth. Yet, at the frontal face where the interface is more exposed, a predominantly hydrophilic region emerges, as inferred from the protein hydrophilic / lipophilic surface calculated using MOE.

### Assembly of an ERCC1-XPF small molecule inhibitors set

To select and validate the docking protocol, a collection of compounds known for their biological activity against the target was amassed, sourced from the ChEMBL28 database. Literature review reveals numerous studies that have employed the compound designated as F06 (below) as an initial reference.

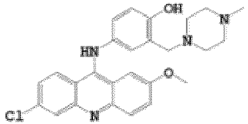

Despite F06 having a recognized yet suboptimal pharmacokinetic and physicochemical profile, it has been frequently utilized as a starting point in various studies due to its ability to disrupt the interaction between ERCC1 and XPF. Notably, F06 interacts within the pocket defined in this study, forming a complex with XPF. The inhibitor set showcases activities from as low as 330 nM to those deemed inactive (activities above 100 µM).

A pool of 84 molecules, known for their activity against ERCC1-XPF, was gathered and subjected to an exhaustive analysis. This centered on diverse properties and descriptors, including molecular weight, H-bond acceptors, H-bond donors, logP, rotatable bonds, heavy atoms, Lipinski violations, among others. Molecular descriptors were computed using FAF-Drugs4 and MOE v.2020.0901, which spanned both conventional and pharmacophore-based descriptors aligned with drug-likeness rules, ideal drug attributes, and medicinal chemistry properties. These molecules exhibit structural diversity, ranging from molecular masses of under 200 g/mol to 600 g/mol, accompanied by a broad spectrum of logP values, suggesting varying lipophilicity levels (Figure 3).

The compound set was employed to develop and validate the docking protocol. Analysis of molecules displaying activity up to 30 µM revealed that the molecular weight primarily ranged between 200 and 400 g/mol, featuring 2 to 3 H-bond donors and 4 or more H-bond acceptors. The LogP values were generally between 1 and 3 with no violations of Lipinski's rule of five. This data is crucial for selecting compounds after virtual screening.

### Optimization of Docking Protocol and Virtual Screening Campaign

To ascertain and validate an optimal docking protocol for subsequent virtual screening campaigns in extensive molecular databases, a series of docking simulations were conducted. Four widely recognized scoring functions available in the GOLD software suite (ChemPLP, GoldScore, ChemScore, and ASP) were assessed. The small molecules from the ChEMBL dataset underwent preprocessing, entailing the retrieval of corresponding SMILES notations, conversion to 3D structures, and preparation under physiological conditions (pH 7.4 and 300 K) using the Molecular Operation Environment (MOE) v.2020.0901. An energy minimization step using the semi-empirical PM7 method ensured an energetically favorable starting conformation.

Each small molecule was then docked into the four complex crystal structures, with three independent systems defined as receptors: complex, XPF, and ERCC1 alone. This used all available scoring functions. The evaluation examined the scoring functions and systems for scoring accuracy, ranking capability, and ligand efficiency. The docking site (center) was defined using a specific set of residues as a reference (performed individually for each residue) within a 15 Å radius. Furthermore, the binding region for the most energetically favorable small molecule conformations was determined to encompass the interaction zone within the complex, specifically the region close to the Phe293 residue.

Upon completion of docking simulations, results were analyzed to establish correlations between each molecule's score and its documented inhibitory activity. The primary goal was to enhance the protocol's ability to distinguish highly active compounds and assign a more accurate score. Using the complex as a system showed poorer performance as the molecules couldn't access the binding region. Docking into individual proteins, the XPF system displayed improved occupancy. Evaluating all molecular docking results for the 84 ChEMBL molecules, the optimal and most reproductible parameters identified and selected involved the GOLD software, the ChemPLP scoring function (with 500 GA runs), docking at the Lys860 residue in XPF, and employing the 1Z00 crystallographic structure (presented the best placement of the molecules into the pocket with the best correlation between score and inhibitory activity comparing all systems).

Following the established docking protocol, the virtual screening process was initiated for the NCI (284,176 compounds), ChemBridge (1,008,227 compounds), and DrugBank (524 compounds) databases, totaling 1,210,397 molecules. These databases of compounds offer a plethora of diverse chemical structures essential for virtual screening, increasing the chances of identifying novel and potent lead compounds. Their diversity ensures a comprehensive exploration of chemical space during virtual screening (various functional groups, scaffolds, and stereochemical configurations).

Compounds from the virtual screening (for each of the databases) were prioritized based on score, ligand efficiency, optimal pocket complementarity (via visual inspection), and interactions with neighboring residues. Results were filtered focusing on score, molecular weight (MW), ligand efficiency / LE), logP, and Pan-assay interference compounds (PAINS) identification. From the top 5,000 molecules scored compounds, those exceeding a score of 90 underwent further descriptor-based filtering, visual inspection, and clustering of structurally similar scaffolds using Murcko Scaffold decomposition in RDKit. The similarity was assessed using the Tanimoto coefficient computed over Morgan fingerprints, a method also implemented in RDKit. This extensive evaluation refined the molecules selection for this challenging target, diversifying the scaffold assortment within the chosen set of molecules (Figure 4).

The DrugBank database's compounds (524 approved drugs) underwent systematic screening and prioritization. Compounds with docking scores over 75, lacking PAINS scaffolds, and interacting at the XPF protein's Lys860 or adjacent residues were retained for further investigation. Visual assessment was pivotal in finalizing the selection, resulting in 48 compounds, which were then clustered by structures. Within the DrugBank compound list, five clusters emerged. Cluster 1, presenting the prevalent scaffold, was the most populated with six structures (Figure 4).

This analysis pinpointed different substituents on a shared scaffold. Hence, only Bisoprolol and Betaxolol were selected for further examination due to their docking pose within the pocket and superior scores within this group. After comprehensive data analysis, seven DrugBank compounds were selected for biological evaluation. A similar procedure was applied to assess the NCI and ChemBridge dataset results (see Figure 5 for schematic representation).

In the context of screening the NCI database, 284,176 compounds underwent preliminary screening. The top 5,000 compounds exhibiting the highest scores were shortlisted for further analysis. These compounds were further assessed, commencing with a filtration step where only those with score exceeding 90 were retained; this threshold matches the score of the best inhibitors from the ChEMBL dataset (Figure 5). Ultimately, a set of 801 compounds were visually inspected, leading to the selection of a final set of 95 compounds for acquisition. Of these 95 compounds, only 35 were available through the NCI platform and were subsequently ordered for experimental testing.

In this dataset, 13 clusters were identified. Both clusters 1 and 2 contained 10 molecules each. One important note is that the most representative scaffold determined via the Tanimoto coefficient may not always be relevant for comparative analysis of the compounds, especially when other groups besides the shared scaffold differ significantly. Cluster 3 identified a secondary amide substituted with two alkyl chains. Upon examination of all structures within this cluster, substantial diversity among the nine molecules was observed. Consequently, relying solely on this analysis to guide compound selection may not be feasible.

For the ChemBridge database's virtual screening, a total of 1,008,227 compounds were screened using a workflow similar to the one applied for the NCI database. The top 5,000 compounds with the highest scores were kept. In this dataset, 25 clusters were identified, with cluster 1 comprising 30 molecules sharing the same scaffold. Molecules from each cluster underwent visual inspection in relation to their pocket placement, guiding compound selection accordingly. All chosen molecules presented scores above 90. A final subset of 64 compounds was identified and subsequently purchased.

In summary, throughout the three virtual screening campaigns targeting the XPF protein, a total of 106 compounds were selected for subsequent biological evaluation. These were sourced from the NCI (35 compounds), ChemBridge (64 compounds), and DrugBank (7 compounds) databases.

One of the primary objectives of this drug design protocol was to identify compounds with diverse chemical profiles for evaluation. To assess this diversity, the chemical space to each final dataset of selected compounds was mapped, as illustrated in Figure 6. This plot depicts the chemical space of the compounds from ChEMBL with activities either greater or lesser than 30 nM, alongside those selected from the NCI, Chembridge, and DrugBank. Notably, there is a discernible dispersion of compounds when comparing those selected from the virtual screening to those from ChEMBL. Compounds from ChEMBL cluster more closely together, predominantly because their structures are often modifications of the F06 inhibitor. Conversely, the virtual screening campaigns described here employed structures without any specific structural constraints tied to this inhibitor or other active compounds.

In figure 7, the potential inhibitors selected from each database are compared with the position of Phe293 residue from ERCC1 within the XPF pocket. Both compounds and the residue participate in a molecular interaction with the Lys860 residue from XPF protein. The compounds from NCI (labeled C) and ChemBridge (labeled D) orient their aromatic moieties towards the upper part of the pocket, effectively occupying the entrance to the pocket. Conversely, the compound from DrugBank, Bisoprolol, positions its primary alkyl primary amine group in the same region as the aromatic rings of the other compounds. It is noteworthy that the Phe293 residue also displays an aromatic side chain in this same region.

### Characterization of NSCLC cell lines

### ERCC1 and ERCC4 gene expression in NSCLC cell lines

To evaluate the basal levels of ERCC1-XPF, attributing a NER status to each NSCLC cell line, mRNA levels of the desired genes were quantified using qRT-PCR. The ERCC 1 gene translates into the ERCC1 protein, forming a complex with the XPF endonuclease, represented by the ERCC4 gene.

Of all the studied cell lines, H1299 presented the highest ERCC1 expression (Figure 8 A and 8 C, respectively). This cell line was followed by A549 cells in terms of highest ERCC 1 expression. Also, for ERCC1 expression, H1975, HCC827, and H1993 cells presented the lowest value. There was no statistical significance between all cell lines in what regards to ERCC4 expression (Figure 8 B).

The results for ERCC1 expression are in accordance with Cai et al. These authors also observed a higher expression for H1299 cells, followed by the A549 cell line. It was also reported that H1975 and HCC827 cells presented the lowest levels of ERCC1, being in the same range.

Cheng et al. also obtained the same results regarding A549 and HCC827 cells, with A549 cells having the highest expression. In what concerns the results of the present invention showing that H1299 cells have the highest expression of ERCC1, He et al. also corroborated this high expression pattern. In view of this, the H1299 cell line was selected for the following cell-based assays.

### Cisplatin cytotoxicity profile in H1299 cells

To evaluate the cisplatin cytotoxicity effect in the selected H1299 cell line (highest ERCC1 expression), a concentration-response profile was established resorting to two mechanistically different cell viability assays, the CV staining and the MTS reduction assays (Figure 9). The IC₅₀ values for cisplatin were also calculated for a 72-h incubation period based on its dose-response profile.

Cell viability decreased in a concentration-dependent manner and the profiles were similar for both assays. Consequently, H1299 cells presented IC₅₀ values of 3 µM and 3.7 µM based on the results from the CV and MTS assays, respectively.

### Validation of the putative ERCC1-XPF inhibitors in in vitro cell-based assays

### Screening of the putative ERCC1-XPF inhibitors in combination with cisplatin on H1299 cells

The next step was to verify if the selected inhibitors do indeed enhance the cytotoxicity induced by cisplatin. With this objective in mind, an MTS assay was performed as a measure of cell viability. H1299 cells were selected for these assays since these cells displayed the highest expression of ERCC1, being thus adequate as a model to mimic a clinical situation present in a subset of patients whose tumors overexpress this NER component. Further tests were performed to assess the cytotoxic effects of putative ERCC1-XPF inhibitors in human non-tumoral cell lines of lung (BEAS-2B) and kidney (HK-2) (see Figure 14).

In short, and since cisplatin alone at 1 µM induces between 10-15% of cell death in H1299 cells (see Figure 9), cells were incubated with both cisplatin (1 µM) and each putative inhibitor (50 µM) for 72h. This concentration of cisplatin was selected since it encompasses only a slight decrease in cell viability due to cisplatin effect alone thus enabling to observe the enhancement of the cytotoxic effects promoted by the putative inhibitor upon combination.

It should however be noted that this concentration is enough to induce cytotoxicity in values higher than the normal scale of error among cell-based methodologies (up to 5-10%). As for the putative inhibitors, the concentration of 50 µM was selected since it represents a balanced compromise between two major aspects that should be considered, i.e. efficacy as NER inhibitors vs inherent compound toxicity.

In fact, if higher concentrations were selected (e.g. 100 µM) it is plausible that a larger number of compounds would be cytotoxic *per se* due to their inherent toxicological properties. However, if this concentration level is decreased at the initial screening phase, a number of interesting compounds that function only at high concentrations might be lost.

The results for all compounds selected from the ChemBridge, NCI, and DrugBank databases are depicted in Figure 10. To note that one compound (CB41) of the 64 compounds ordered from ChemBridge was not available and 3 compounds (NCI2, NCI8, and NCI23) from NCI were not soluble in DMSO, precluding its evaluation in the cell-based assays. For this reason, a total of 102 molecules were evaluated.

With the purpose of selecting the best inhibitors among the pool of 102 tested compounds, a decision-making process was systematically employed. This strategy is depicted in Figure 11. In short, the first step was to evaluate the impact of the combination of cisplatin with the putative inhibitor in comparison with cisplatin alone in H1299 cells.

To achieve this, the cell viability presented by the combined condition relative to cisplatin viability was calculated, and if inferior to 85%, the compounds moved to the next stage. The use of this threshold identifies compounds that increase cisplatin cytotoxicity by at least 15%. From this step, 46 compounds were discarded (i.e., 29 from CB, 4 from DB, and 13 from NCI). The next step addressed the fact that some putative inhibitors are indeed highly cytotoxic *per se.* In fact, while some of them completely abolished cisplatin viability upon combination, some were concomitantly toxic alone. So, if the compounds alone at 50 µM induced more than 70% cytotoxicity (i.e., presented a viability < 30%), they were removed at this stage from the pool of best inhibitors. Nevertheless, these compounds were not yet discarded but rather moved to a next approach that consists of lowering the concentration tested in cells to 10 µM (5-fold, as results shown in Figure 12).

As a result, 23 compounds were taken out of the pool of top compounds (9 from CB, 2 from DB, and 12 from NCI). From the remaining 33 putative inhibitors, a next decision step was introduced to consider the influence directly ascribed to the putative inhibitor in the combinatory effect and not merely the result of its own cytotoxicity in NSCLC cells.

To verify this, the difference between the viability of the inhibitor alone was subtracted to the combinatory condition. This information allowed the calculation of the inhibitor effect, and if it was higher than 10%, it was considered in the short list of the best inhibitors. The resulting best inhibitors were 15 from ChemBridge, 1 from DrugBank and 6 from NCI databases (total of 22 compounds), to be used in further studies.

### Extremely cytotoxic compounds in H1299 cells

Since some compounds demonstrated to be extremely cytotoxic to NSCLC cells (cell viability less than 30%), a further step was performed by lowering the concentration applied to the cells (Figure 12). In this additional assay, a concentration of 10 µM was selected for each compound to be combined with 1 µM of cisplatin, and a total of 23 compounds were tested (9 from ChemBridge, 2 from DrugBank, and 6 from NCI databases).

Even with the strategy of reducing the concentration of the extremely cytotoxic compounds to 10 µM, there were still five compounds that induced more than 50% of cell death. Nevertheless, none of the other 18 compounds could enhance cisplatin cytotoxicity in a significant manner. For this reason, they were not selected for further studies.

### Computational Simulations

### Selection and Preparation of Protein Structures

The ERCC1-XPF endonucleases complex was identified as a potential target for NER inhibitors. As of the time of this study, eight three-dimensional structures of the ERCC1-XPF complex were accessible on the Protein Data Bank (PDB) (as disclosed in Table 1). Among these, none featured small-molecule inhibitors, and only one showed the complex interacting with DNA. All the crystal structures available represent the human variant of the complex.

The present analysis focused on four structures that showcase the complex of the C-terminal domains of human XPF and ERCC1, namely: 1Z00, 2A1J, 6SXA, 6SXB. These complex structures were retrieved from the PDB database. The preparation of each crystal structure was undertaken using the Molecular Operating Environment (MOE v2020.01) structure preparation module. Hydrogen atoms were added, and appropriate protonation states were assigned using Protonate-3D tool at pH 7.4 within the MOE software package v2020.0901. Crystallographic water molecules and other non-relevant entities not associated with the ERCC1, and XPF structures were excised.

### Selection and preparation of the ligands

A comprehensive search of the ChEMBL database was conducted to retrieve a dataset of known small molecules inhibitors targeting ERCC1-XPF, wherein 84 entries were identified. The SMILES notation of these compounds was procured and subsequently converted into 3D structures.

Each molecule was prepared using the Protonate 3D tool within MOE v.2020, at pH 7.4 and 300 K. Subsequently, the molecules underwent energy minimization employing using the PM7 semi-empirical method to ensure an optimal starting conformation. The ligands' physicochemical properties were assessed using both MOE software and FAF-Drugs4 webserver.

### Validation of Docking and Virtual Screening Approaches

To validate the docking methodology used in the present invention, benchmark studies were carried out using GOLD v5.07 software package. Four scoring functions were assessed: ChemPLP, GoldScore, ChemScore, and ASP. Each identified small molecule, termed as a ligand, was docked onto the available protein crystal structures.

Three different receptor configurations were explored: the complete ERCC1-XPF complex, and XPF or ERCC 1 proteins alone. Independent docking simulations were performed for each ligand, scoring function, and receptor combination.

The docking site was anchored using Lys860 residue and Ile862 residues, both crucial for complex integrity, with a 15 Å radius around them. All scoring functions were assessed for their efficacy in scoring and ranking. Based on the present evaluation, ChemPLP was chosen for subsequent virtual screening efforts, employing 500 GA runs. Three databases were targeted for further virtual screening: NCI (National Cancer Institute Library), ChemBridge (commercial database), and DrugBank (database comprising approved and commercialized drugs).

### Ranking and Assessment of compounds from virtual screening:

Docked-based virtual screening produced compounds that were ranked considering multiple criteria, such as scoring metrics, ligand efficiency, binding pocket fit, and interactions with adjacent residues. A thorough analysis of each compound was conducted, focusing on parameters like molecular weight (MW), hydrogen-bond donors, hydrogen-bond acceptors, rotatable bonds, and logP values.

Compounds targeting Lys860 residue in XPF were visually inspected using the MOE software. Emphasis for top-ranking molecules was on their pose within the binding pocket. Compounds exhibiting potential PAINS interactions were systematically eliminated.

### Determination of Key interactions, Clustering Structurally Similar Scaffolds and Chemical space

The Murcko Scaffold decomposition method, which is available in RDKit, was used to cluster structurally similar scaffolds. The similarity between these scaffolds was gauged by calculating the Tanimoto coefficient, specifically utilizing Morgan fingerprints, also implemented in the RDKit. The RDKit was also used to calculate 2D and 3D physicochemical descriptors. Visual clustering was performed with the t-SNE dimensionality reduction, using the function implemented in scikit-leam (TSNE function) projecting the original 1024 dimensions into two final dimensions.

The PDB files for each studied system were processed using the PLIP (Protein-Ligand Interaction Profiler) algorithm, through the PLIP python module. This tool examines and visualizes protein-ligand interactions in PDB files, facilitating a comparative assessment of interactions across systems. The present analysis considered various interactions, including H-bonds, hydrophobic interactions, π-stacking, water bridges, halogen bonds, and salt bridges.

### Cell-based Assays

### Chemicals

RPMI-1640 with L-glutamine was purchased from Biowest (Nuaillé, France). Cisplatin, fetal bovine serum (FBS), penicillin-streptomycin solution (10,000 units/mL of penicillin; 10 mg/mL of streptomycin), crystal violet (CV), trypsin (0.25%), sodium bicarbonate, and dimethylsulfoxide (DMSO) were purchased from Sigma-Aldrich (Madrid, Spain). Sodium pyruvate was purchased from Lonza (Switzerland). HEPES and D-Glucose were purchased from AppliChem (Darmstadt, Germany).

A 2 mM stock solution of cisplatin was prepared in saline (0.9% NaCl), aliquoted and stored at -20 °C. Further diluted stock solutions were also prepared in saline, aliquoted and stored at -20 °C.

The acquired compounds from the Chembridge, NCI and DrugBank databases were reconstituted in 100% DMSO at a concentration of 12.5 mM, aliquoted and stored at -20 °C. A further dilution to 2.5 mM was also performed in 100% DMSO and stored at -20 °C, when needed. Working solutions of each compound were freshly diluted in complete cell culture medium so that, in each final solution, the DMSO concentration was kept at 0.4% (v/v) when in contact with cells.

In all cell-based assays, vehicle-treated controls were also included, in which cells were exposed to the respective solvents, i.e., DMSO (final concentration of 0.4% (v/v)) and/or saline.

### Cell culture

The human NSCLC H1975, A549, H460, H1993, HCC827, and H1299 cell lines were obtained at the American Type Culture Collection (ATCC, Manassas, VA, USA). All cell lines were cultured in monolayer in RPMI-1640 medium with L-glutamine supplemented with 10 mM HEPES, 2.5 g/L D-glucose, 1 mM Sodium pyruvate, 1.5 g/L Sodium bicarbonate, 10% FBS and 1% Pen/Strep (complete cell culture medium) and were maintained at 37 °C, under a humidified atmosphere containing 5% CO₂ in air.

### Gene expression

To select the best NSCLC cell model to study the putative inhibitors, a characterization of the expression of ERCC1 and ERCC4 (XPF) genes in all cell lines was performed by using quantitative reverse transcription PCR (qRT-PCR), following a previously described protocol. Total RNA was isolated, from each cell line (1.0×10⁶ cells/sample), using TRIzol^{™} Reagent (Invitrogen^{™}, Waltham, Massachusetts, EUA) and extracted according to the manufacturer's instructions. RNA concentration was assessed by measuring the absorbance at 260 nm using LVis plate mode (SPECTROstar Omega, BMG Labtech). cDNA was synthesized from 1 µg of RNA using NZY First-Strand cDNA Synthesis Kit (NZYTech, Lisbon, Portugal), according to the manufacturer's instructions. qRT-PCR was performed using PowerUp^{™} SYBR^{®} Green Master Mix (Applied Biosystems^{™}, Waltham, Massachusetts, EUA) for a final reaction volume of 15 µL, using 2 µL of template cDNA and 0.33 µM of forward and reverse primers. The primer sequences used were the following: ERCC1, forward, 5'-CTACGCCGAATATGCCATCTC-3', reverse, 5'-GTACGGGATTGCCCCTCTG -3'; ERCC4 (XPF), forward, 5'-CACCTCCCTCGCCGTGTA-3', reverse, 5'-CGCAAATATAACACCACCTTGTG-3'; β-actin, forward, 5'-CATGTACGTTGCTATCCAGGC-3', reverse, 5'-CTCCTTAATGTCACGCACGAT-3'. The reaction was performed on QuantStudio^{™} 7 Flex Real-Time PCR System (Applied Biosystems) and consisted of a 2-min uracil-N-glycosylase activation step at 50 °C and a denaturation step at 95 °C for 10 min, followed by 40 cycles of a denaturation step of 15 seconds at 95 °C and annealing/extension at 60 °C for 1 min. Finally, a dissociation step was added to determine the melting temperature of a single target sequence as a measure of quality and specificity. Relative expression of the two target genes in each cell line was calculated using the expression 2-ΔCt, where ΔCt = Average Ct (gene of interest) - Average Ct (β-ACTIN) and normalized it to the reference gene β-ACTIN. qRT-PCR reactions were performed on duplicate samples of cDNAs from three independent collections of all cell lines.

### Crystal violet staining assay

The cytotoxicity of cisplatin in H1299 cells was evaluated according to a previously described crystal violet (CV) staining protocol. This assay allows the colorimetric determination of adherent cells by staining their nucleus. First, cells were inoculated at a density of approximately 3×10³ cells/well in 200 µL of complete culture medium in 96-well plates. After adherence (24 h), culture medium was renewed, and the cells were incubated with a range of 1-30 µM of cisplatin for another 72 h. After incubation, cells were washed with warm PBS to remove non-adherent cells (non-viable cells). The adherent cells were then fixated with ice-cold 96% ethanol for 10 min and stained with 0.1% crystal violet in 10% ethanol for 5 min. The plate was then washed with tap water and the stained cells were dissolved in 200 µL of 96% ethanol with 1% acetic acid. Absorbance was measured at 595 nm (OD595) using a SPECTROstar OMEGA microplate reader. Values presented by vehicle-treated control cells (untreated cells) corresponded to 100% of cell viability. Three to five independent experiments were performed with six replicates for each condition in each independent experiment. The IC₅₀ values were calculated based on the obtained concentration-response curves using GraphPad Prism^{®} 9.0 (GraphPad Software, Inc., La Jolla, CA, USA).

### MTS reduction assay

The 3-(4,5-dimethylthiazol-2-yl)-5-(3-carboxymethoxyphenyl)-2-(4-sulfophenyl)-2H-tetrazolium salt (MTS) reduction assay was carried out as a confirmatory assay of cell viability. This methodology is commonly used in in vitro toxicology and is based on the enzymatic reduction of a tetrazolium salt in viable cells by mitochondrial enzymes (NAD-dependent dehydrogenase) with the formation of a colored formazan product. Briefly, after incubation with the compound and removal of complete cell culture medium, cells were washed with warm PBS, followed by the addition of 100 µL of fresh medium and 20 µL of MTS substrate prepared from the CellTiter 96^{®} Aqueous MTS (Promega, Madison, WI, USA) according to the manufacturer's instructions. H1299 cells were incubated for 2h30 with the MTS reagent and the absorbance was measured at 490 nm and 690 nm (reference wavelength) using a SPECTROstar OMEGA microplate reader. Absorbance values presented by vehicle-treated control cells corresponded to 100% of cell viability. Two to four independent experiments were performed, and three replicates were used for each condition in each independent experiment. The IC₅₀ was also calculated based on the concentration-response curve using GraphPad Prism^{®} 9.0.

### First screening approach of the ERCC1-XPF inhibitors in a NSCLC cell line

The compounds previously identified as putative ERCC1-XPF inhibitors were first evaluated in a set of combinatory cell-based experiments with cisplatin. A concentration of 50 µM of each compound was combined with 1 µM of cisplatin and applied to H1299 cells (highest ERCC1 expression). The MTS reduction assay was performed, as previously described, for the assessment of cell viability, and a 72-h incubation period was conducted for both compounds. For the compounds which were considered to be highly cytotoxic in the first approach, a further assay was performed in the same cell line by lowering their concentration to 10 µM. Two to three independent experiments were performed.

### Statistical analysis

The results are presented as Average ± SD. Data was analyzed with one-way ANOVA multiple comparisons with GraphPad Prism^{®} 9.0. p < 0.05 was considered statistically significant (represented as: *p < 0.05, **p < 0.01, ***p < 0.001, and ****p < 0.0001).

The above-described subject matter is provided as an illustration of this invention and should not be interpreted in a limiting sense. The terminology used to describe preferred embodiments of this invention should not be construed to limit the invention to those particular embodiments.

As used in the description, definite and indefinite articles, in their singular form, are intended to encompass plural forms unless the context of the description explicitly indicates otherwise.

The indefinite articles "a" or "an" should generally be interpreted as "one or more" unless the sense of a singular embodiment is clearly defined in a specific situation.

It will be understood that the terms "comprise" and "include", when used in this description, specify the presence of stated features, elements, components, steps, and operations but do not preclude the presence or addition of one or more other features, elements, components, steps, and operations.

As used throughout this patent application, the term "or" is used in an inclusive sense rather than an exclusive sense unless the exclusive sense is clearly defined in a specific situation. In this context, a phrase such as "X utilizes A or B" should be interpreted as including all relevant inclusive combinations.

As understood in the art, the term "optionally substituted" indicates that the specified group is either unsubstituted or substituted by one or more suitable substituents. A "substituent" that is "substituted" is an atom or group which takes the place of a hydrogen atom on the parent chain or cycle of an organic molecule.

"Cyclyl" as used herein refers to a cyclic structure within a chemical compound. This term denotes a monovalent radical or substituent derived from a cyclic compound, where one hydrogen atom has been removed to create a point of attachment for further chemical bonding. It includes both carbocyclic (entirely carbon-based rings) and heterocyclic (rings containing non-carbon atoms, such as nitrogen, oxygen, or sulfur) structures.

"Aryl" as used herein refers to a ring system having one or more aromatic rings. Representative examples of aryl include azulenyl, indanyl, indenyl, naphthyl, phenyl, tetrahydronaphthyl, mesityl, thienyl, and the like.

"Heteroaryl" means a cyclic, aromatic hydrocarbon in which one or more carbon atoms have been replaced with heteroatoms (e.g., N, O or S). If the heteroaryl group contains more than one heteroatom, the heteroatoms may be the same or different.

"Hydrogen bond donor" as used herein means a group that under suitable conditions can ionize to release a H+ ion and to produce a negatively charged species. Examples of groups of this type include inorganic acids, sulfonic acids, carboxylic acids, anionic amino acids, hydroxyl acids, fatty acids for insoluble salts.

"Hydrogen bod acceptor" as used herein means a group that under suitable conditions can react with H+ ion to form a positively charged species. Examples of groups of this type include organic amines, cationic amines and bases for insoluble salts.

"Alkoxy" as used herein, refers to an alkyl group, as defined herein, appended to the parent molecular moiety through an oxy group, as defined herein.

An "amine" or "amino" is intended to mean the group -NH₂. "Primary amines" have one of three hydrogen atoms replaced by an alkyl or aromatic group. "Secondary amines" have two organic substituents bound to the nitrogen together with one hydrogen.

An "amide" as used herein refers to a functional group having a carbonyl group (C=O) linked to a nitrogen atom (N), or an organic compound that contains this group.

The term "oxo" as used herein, refers to a =O moiety. The term "oxy" as used herein, refers to a -O- moiety.

"Nitro" refers to the organic compound functional group -NO₂.

"Carbonyl" is a functional group having a carbon atom double bonded to an oxygen atom (-C=O).

"Carboxy" as used herein refers to a -COOH functional group, also written as -CO₂H or -(C=O)-OH.

All changes, as long as they do not modify the essential characteristics of the following claims, should be considered within the scope of protection of this invention.

### Citation List

The citation list is as follows:

### Patent Literature

PTL1: CN111298122B

### Non-Patent Literature

NPL1: Gentile, F., Barakat, K., & Tuszynski, J. (2018). Computational Characterization of Small Molecules Binding to the Human XPF Active Site and Virtual Screening to Identify Potential New DNA Repair Inhibitors Targeting the ERCC1-XPF Endonuclease. International Journal of Molecular Sciences, 19(5), 1328. doi:10.3390/ijms19051328
NPL2: Jones, M.; Beuron, F.; Borg, A.; Nans, A.; Earl, C.P.; Briggs, D.C.; Snijders, A.P.; Bowles, M.; Morris, E.P.; Linch, M.; et al. Cryo-EM structures of the XPF-ERCC1 endonuclease reveal how DNA-junction engagement disrupts an auto-inhibited conformation. Nat. Commun. 2020, 11, 1-14.
NPL3: Faridounnia, M.; Wienk, H.; Kovačič, L.; Folkers, G.E.; Jaspers, N.G.J.; Kaptein, R.; Hoeijmakers, J.H.J.; Boelens, R. The cerebro-oculo-facio-skeletal syndrome point mutation F231L in the ERCC1 DNA repair protein causes dissociation of the ERCC1-XPF complex. J. Biol. Chem. 2015, 290, 20541-20555.
NPL4: Tsodikov, O. V.; Enzlin, J.H.; Schärer, O.D.; Ellenberger, T. Crystal structure and DNA binding functions of ERCC1, a subunit of the DNA structure-specific endonuclease XPF-ERCC1. Proc. Natl. Acad. Sci. U. S. A. 2005, 102, 11236-11241.
NPL5: Das, D.; Folkers, G.E.; Van Dijk, M.; Jaspers, N.G.J.; Hoeijmakers, J.H.J.; Kaptein, R.; Boelens, R. The structure of the XPF-ssDNA complex underscores the distinct roles of the XPF and ERCC1 helix-hairpin-helix domains in ss/ds DNA recognition. Structure 2012, 20, 667-675.
NPL6: Das, D.; Tripsianes, K.; Jaspers, N.G.J.; Hoeijmakers, J.H.J.; Kaptei, R.; Boelens, R.; Folkers, G.E. The HhH domain of the human DNA repair protein XPF forms stable homodimers. Proteins 2007, 70, 1551-1563.
NPL7: Tripsianes, K.; Folkers, G.; Ab, E.; Das, D.; Odijk, H.; Jaspers, N.G.J.; Hoeijmakers, J.H.J.; Kaptein, R.; Boelens, R. The structure of the human ERCC1/XPF interaction domains reveals a complementary role for the two proteins in nucleotide excision repair. Structure 2005, 13, 1849-1858.
NPL8: Cai, Y.; Yan, X.; Zhang, G.; Zhao,W.; Jiao, S. The predictive value of ERCC1 and p53 for the effect of panobinostat and cisplatin combination treatment in NSCLC. Oncotarget 2015, 6, 18997-19005
NPL9: Cheng, H.; Zhang, Z.; Borczuk, A.; Powell, C.A.; Balajee, A.S.; Lieberman, H.B.; Halmos, B. PARP inhibition selectively increases sensitivity to cisplatin in ERCC1-low non-small cell lung cancer cells. Carcinogenesis 2013, 34, 739-749.
NPL10: He, Y.; Chen, D.; Yi, Y.; Zeng, S.; Liu, S.; Li, P.; Xie, H.; Yu, P.; Jiang, G.; Liu, H. Histone Deacetylase Inhibitor Sensitizes ERCC1-High Non-small-Cell Lung Cancer Cells to Cisplatin via Regulating miR-149. Mol. Ther. - Oncolytics 2020, 17, 448-459.

## Claims

1. IERCC1-XPF complex inhibitors compounds comprising:
at least one optionally substituted cyclyl, heterocyclyl, aryl or heteroaryl group;
from 2 to 3 hydrogen bond donors selected from at least one of a hydroxyl group, a thiol group, a fluoride group and a nitrogen-containing group;
at least 4 hydrogen bond acceptors selected from at least one of oxygen, nitrogen, sulfur and halogens;
wherein the molecular weight ranges between 200 and 400 g/mol;
and / or a pharmaceutically acceptable salt, solvate or tautomer thereof for use as a medicament in the treatment of cancer, tumors and / or metastases, wherein the compound and the Phe293 residue from ERCC1 participate in a molecular interaction to destabilize the ERCC1-XPF complex.

2. ERCC1-XPF complex inhibitors compounds comprising:
at least one optionally substituted phenyl, furanyl, pyrrolyl, thienyl, oxazolyl, indazolyl, isoxazolyl, thiazolyl, isothiazolyl, imidazolyl, pyrazolyl, triazolyl, pyridyl, pyridinyl, dihydrothiadiazolyl, dioxazolyl, oxadiazolyl, pyrazinyl, pyrimidinyl, pyridazinyl, piperazinyl, triazinyl, indolyl, quinolinyl, isoquinolinyl, purpyrrolyl, benzimidazolyl, benzoxazolyl, benzothiazolyl, pteridinyl, azepanyl, diazinanyl, pyrrolidinyl, imidazolidinyl, morpholinyl or other mono- or poly aromatic or non-aromatic homocyclic or heterocyclic compound;
from 2 to 3 hydrogen bond donors selected from at least one of a hydroxyl group, a thiol group, a fluoride group and a nitrogen-containing group selected from substituted or unsubstituted amino group selected from primary or secondary amine or amide;
at least 4 hydrogen bond acceptors selected from at least one of oxygen, nitrogen, sulfur and halogens, the oxygen being found in carbonyl groups, hydroxyl groups, ether groups or esters groups, the nitrogen being found in amines, amides and heteroaryl groups, and the sulfur being found in sulfonyl and thiocarbonyl groups;
wherein the molecular weight ranges between 200 and 400 g/mol;
and / or a pharmaceutically acceptable salt, solvate or tautomer thereof for use, according to claim 1, as a medicament in the treatment of cancer, tumors and / or metastases, wherein the compound and the Phe293 residue from ERCC1 participate in a molecular interaction to destabilize the ERCC1-XPF complex.

3. ERCC1-XPF complex inhibitor compounds selected from:
N-{3-methoxy-4-[2-(2-pyridinyl)ethoxy]benzyl}-N-[(3S)-2-oxo-3-azepanyl]-2-[3-(trifluoromethyl)phenyl]acetamide 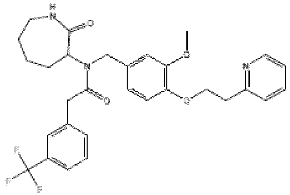
1-benzyl-N'-cyclopropyl-N-methyl-4-oxo-N-[(4-phenyl-1,3-thiazol-2-yl)methyl]-1,4-dihydro-3,5-pyridinedicarboxamide 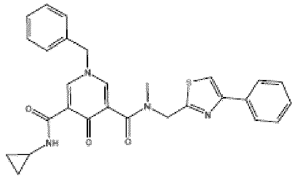
1-[(3,4-dimethoxybenzyl)oxy]-3-[4-(diphenylmethyl)-1-piperazinyl]-2-propanol dihydrochloride 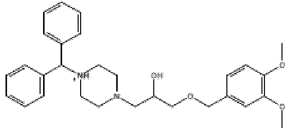
N~2~-benzyl-N~1~-[2-(3,4-dimethoxyphenyl)ethyl]-N~2~-[(4-methoxyphenyl)sulfonyl]glycinamide 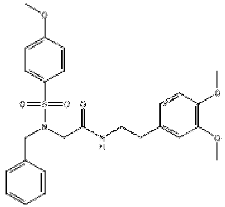
N-{[5-[(cyclohexylmethyl)thio]-4-(4-fluorophenyl)-4H-1,2,4-triazol-3-yl]methyl}-3-(3-methyl-1H-pyrazol-1-yl)propenamide 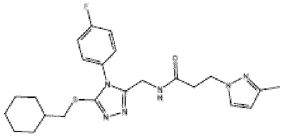
2- f 1-(4-chlorophenyl)-5-oxo-3-[2-(2-thienyl)ethyl]-2-thioxo-4-imidazolidinyl}-N-(4-fluorophenyl)acetamide 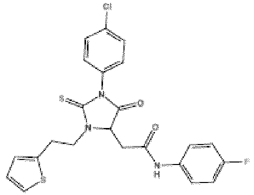
N-(3-ethoxypropyl)-2-[2-[(4-phenyl-1-piperazinyl)methyl]-4-(3-thienyl)phenoxy]acetamide 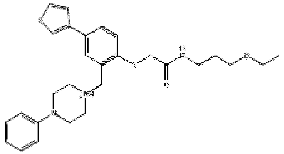
3-[2-(2-naphthylmethyl)-5-oxo-2-pyrrolidinyl]- N-[1-(1-phenyl-1H-pyrazol-4-yl)ethyl]propenamide 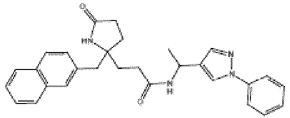
N~2~-benzyl-N~1~-[2-(1-cyclohexen-1-yl)ethyl]-N~2~-[(4-fluorophenyl)sulfonyl]glycinamide 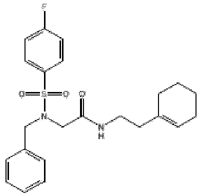
N~2~-benzyl-N~2~-[(4-fluorophenyl)sulfonyl]-N~1-[3-(trifluoromethyl)phenyl]glycinamide 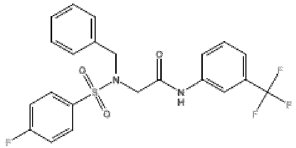
N~2~-benzyl-N~2~-(2,3-dihydro-1,4-benzodioxin-6-ylsulfonyl)-N~1~-[2-(3,4-dimethoxyphenyl)ethyl]glycinamide 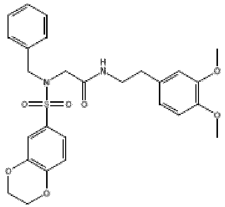
N~2~-benzyl-N~2~-[(3,4-dimethoxyphenyl)sulfonyl]-N~1~-[2-(4-methoxyphenyl)ethyl]glycinamide 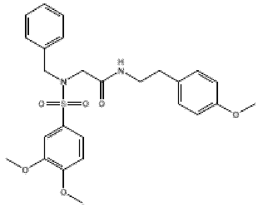
2-{1-(4-chlorophenyl)-5-oxo-3-[2-(4-pyridinyl)ethyl]-2-thioxo-4-imidazolidinyl}-N-(4-methoxyphenyl)acetamide 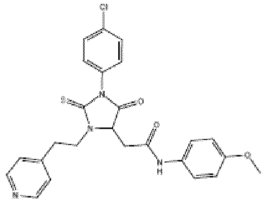
4-{[N-(mesitylsulfonyl)-N-(2-phenylethyl)glycyl]amino}benzamide 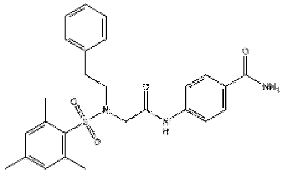
N-[3-(1H-indazol-1-yl)propyl]-3-[5-(1H-indol-3-ylmethyl)-1,3,4-oxadiazol-2-yl]propenamide 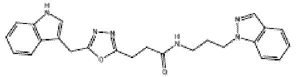
(2R)-2-[(R)-(2-ethoxyphenoxy)-phenylmethyl]morpholine 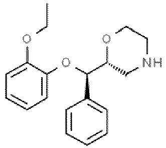
Benzyl 4-methyl-2-[[2-[[2(phenylmethoxycarbonylamino)acetyl]amino]acetyl]amino]pentanoate 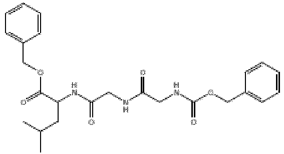
Benzyl 3-phenyl-2-[[2-[[2-(phenylmethoxycarbonylamino)acetyl]amino]acetyl]amino]propanoate 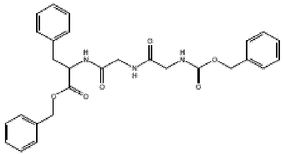
3-Benzyl-1-[3-(3-benzyl-4-oxo-2-sulfanylidene-1,3-diazinan-1-yl)propyl]-2-sulfanylidene-1,3-diazinan-4-one 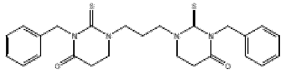
Carbobenzyloxyvalylglycylglycine benzyl ester 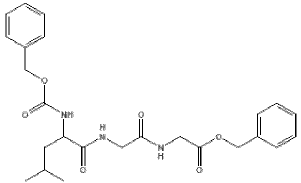 2-acetamido-N-benzyl-6-[benzyl(2-chloroethylcarbamoyl)amino]hexanamide 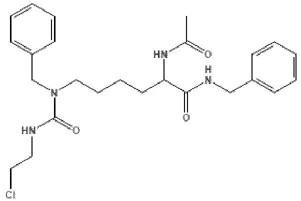
2-[2-[2-(2-phenoxyethoxy)ethoxy]ethoxy]-N-[2-[2-[2-(2-phenoxyethoxy)ethoxy] ethoxy] ethyl] ethanamine 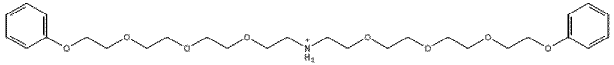
and / or a pharmaceutically acceptable salt, solvate or tautomer thereof for use, according to any of claims 1-2, as a medicament in the treatment of cancer, tumors and / or metastases,
wherein the compound and the Phe293 residue from ERCC1 participate in a molecular interaction to destabilize the ERCC1-XPF complex.

4. ERCC1-XPF complex inhibitors compounds and / or pharmaceutically acceptable salt, solvate or tautomer thereof for use in the treatment of cancer, tumors and / or metastases according to any of claims 1-3 in combination with at least one anticancer agent.

5. ERCC1-XPF complex inhibitors compounds and / or pharmaceutically acceptable salt, solvate or tautomer thereof for use in the treatment of cancer, tumors and / or metastases according to claim 4 wherein the anticancer agent is a platinum-based anticancer agent selected from the group consisting of cisplatin, carboplatin, oxaliplatin, nedaplatin, lobaplatin, satraplatin, and heptaplatin.

6. ERCC1-XPF complex inhibitors compounds and / or pharmaceutically acceptable salt, solvate or tautomer thereof for use according to any of claims 1-5 wherein the cancer is non-small cell lung cancer.

7. Pharmaceutical composition comprising an effective amount of at least one ERCC1-XPF complex inhibitors compound according to any of claims 1-3 and / or a pharmaceutically acceptable salt, solvate or tautomer thereof and excipients.

8. Pharmaceutical composition, according to claim 7, further comprising at least one anticancer agent.

9. Pharmaceutical composition, according to claim 8, wherein the anticancer agent is a platinum-based anticancer agent selected from the group consisting of cisplatin, carboplatin, oxaliplatin, nedaplatin, lobaplatin, satraplatin, and heptaplatin.

10. Kit comprising separate packs of
(a) an effective amount of a ERCC1-XPF complex inhibitors compounds according to any of claims 1-3 and / or a pharmaceutically acceptable salt, solvate or tautomer thereof, and
(b) an effective amount of at least one anticancer agent, wherein the anticancer agent is a platinum-based anticancer agent selected from the group consisting of cisplatin, carboplatin, oxaliplatin, nedaplatin, lobaplatin, satraplatin, and heptaplatin.
